# FASCICULE DE BREVET EUROPEEN

(11) **EP 2 378 980 B1**
(45) Date de publication et mention de la délivrance du brevet: **12.02.2020**
(21) Numéro de dépôt: 09796715.2
(22) Date de dépôt: 18.12.2009
(51) Int. Cl.: A61B 10/00, A01K 11/00

(54) **DISPOSITIF DE PRELEVEMENT DE TISSU D'UN ANIMAL**
VORRICHTUNG ZUR ENTNAME VON MATERIALPROBEN VON EINEM TIER
DEVICE FOR SAMPLING TISSUE FROM AN ANIMAL

(30) Priorité: 19.12.2008 FR 0807265
(43) Date de publication de la demande: 26.10.2011
(73) Titulaire: Allflex Europe, 35500 Vitre (FR)
(72) Inventeur: HILPERT, Jean-Jacques, 35500 Vitre (FR); DECALUWE, Johan, 53000 Laval (FR)
(74) Mandataire: Vidon Brevets & Stratégie
(86) Numéro de dépôt international: PCT/EP2009/067591
(87) Numéro de publication internationale: WO 2010/070130

(56) Documents cités:
- EP-A- 1 504 722
- EP-A- 1 772 104
- EP-A1- 1 366 715
- EP-A1- 1 606 606
- EP-A1- 2 223 656
- WO-A-2007/087261
- WO-A1-2005/079562
- WO-A1-2008/055690
- WO-A2-2004/113874
- FR-A- 2 831 389
- FR-A1- 2 917 574
- GB-A- 2 358 061
- US-A1- 2010 215 548
- US-B1- 6 565 814
- US-B1- 6 659 338
- US-B2- 8 303 914

## Description

### 1. Domaine de l'invention

Le domaine de l'invention est celui du contrôle et/ou de l'identification des animaux.

Plus précisément, l'invention concerne le prélèvement de tissu d'un animal, permettant notamment de conserver des cellules portant des caractéristiques biologiques ou biochimiques de l'animal, par exemple pour identifier ultérieurement l'animal ou détecter des maladies de l'animal. De tels prélèvements sont par exemple effectués lors de la mise en place d'un dispositif de marquage, encore appelé marque.

L'invention permet notamment le prélèvement de tissu sur des bovins, des ovins, des porcins, des caprins, des volatiles, des poissons ou plus généralement sur toute espèce animale.

### 2. Art antérieur

Afin d'améliorer le suivi du cheptel et garantir l'origine des animaux destinés notamment à la consommation, par exemple en détectant des maladies, il est de plus en plus souvent procédé à un prélèvement de tissu des animaux concernés.

Un tel prélèvement peut être effectué directement sur l'animal, à sa naissance par exemple, ou lors de la pose d'une marque d'identification de l'animal.

Une fois collecté, l'échantillon de tissu de l'animal peut être stocké et/ou transmis à un laboratoire pour analyse.

Pour prélever un échantillon lors de la pose d'une marque d'identification sur un animal, on utilise classiquement une marque comprenant une partie mâle présentant une tige creuse et une partie femelle, destinée à être recevoir au moins partiellement la tige. La tige creuse permet le passage d'un poinçon, qui peut être retiré après la mise en place de la marque. Un tel poinçon présente une arête coupante permettant de perforer la peau de l'animal lors de la mise en place de la marque.

Ainsi, lors de l'emboîtement des parties mâle et femelle, le poinçon perfore la peau de l'animal le long d'une ligne de contact, et guide la tige dans la partie femelle. L'échantillon de tissu découpé par l'arête coupante reste emprisonné dans le poinçon. Après retrait du poinçon, l'échantillon peut être extrait du poinçon et stocké. WO-A-2005/079562 divulgue un tel dispositif. L'échantillon peut également être conservé quelque temps dans le poinçon.

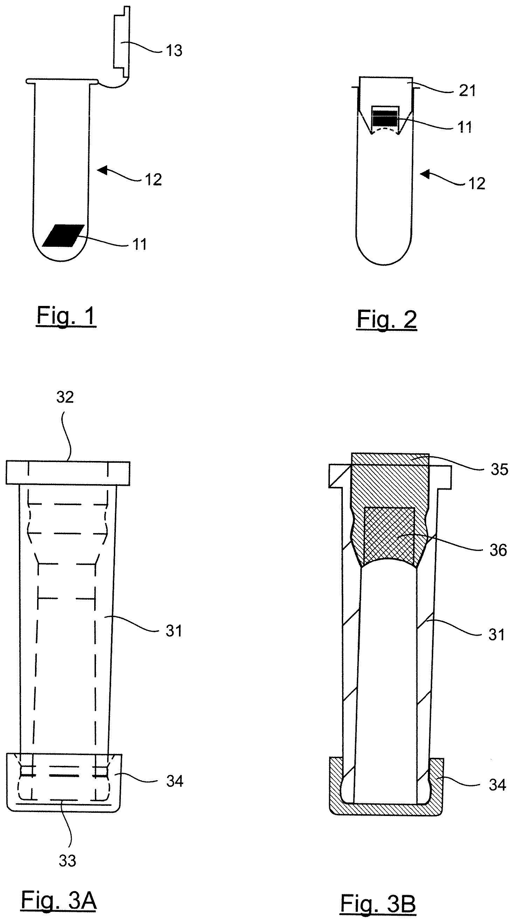

Par exemple, comme illustré en figure 1, un échantillon de tissu 11 prélevé sur un animal peut être stocké dans un microtube 12 en forme de capuchon. Un tel microtube 12 peut être fermé par un couvercle 13, éventuellement relié au microtube 12.

Un agent conservateur peut également être introduit dans le microtube 12, permettant notamment de sécher l'échantillon et d'assurer sa conservation sur une longue durée, de plusieurs mois à plusieurs années par exemple.

Un inconvénient de cette technique est que les opérations d'extraction de l'échantillon 11 du poinçon et d'insertion dans le microtube 12 sont fastidieuses. En particulier, il existe un risque de contamination de l'échantillon lors de ces opérations.

D'autres techniques de prélèvement ont également été proposées, permettant d'insérer directement l'échantillon dans le microtube lors du prélèvement. Ces techniques peuvent être mises en œuvre indépendamment de la pose de la marque, ou conjointement à la pose de la marque.

Comme illustré en figure 2, ces techniques reposent sur l'utilisation d'un emporte-pièce formant ou comprenant un élément de coupe 21, destiné à découper un échantillon 11 de tissu de l'animal et à le collecter dans un logement. L'emporte-pièce 21, fixé à une première mâchoire d'un outil de prélèvement comme une pince par exemple, vient découper la peau de l'animal, et s'insérer au moins partiellement dans le microtube 12, fixé à une deuxième mâchoire de l'outil, lors de l'actionnement de l'outil. L'emporte-pièce 21 présente classiquement un diamètre légèrement inférieur à celui du microtube 12, de façon à servir de couvercle hermétique au microtube. Le logement recevant l'échantillon 11 est donc ouvert vers l'intérieur du microtube, mais fermé vers l'extérieur du microtube, de façon à éviter une contamination de l'échantillon.

Grâce à ces techniques de prélèvement, l'échantillon 11 est directement inséré dans le microtube 12, limitant ainsi le risque de contamination de l'échantillon.

Un inconvénient de cette technique est qu'une fois l'emporte-pièce 21 inséré dans le microtube 12, il n'est plus possible d'ajouter de produit dans le microtube, comme un agent conservateur ou un réactif, sans avoir à retirer ou découper l'emporte-pièce 21.

Il existe donc un besoin pour une nouvelle technique de prélèvement de tissu d'un animal ne présentant pas l'ensemble de ces inconvénients de l'art antérieur.

### 3. Exposé de l'invention

L'invention propose une solution nouvelle qui ne présente pas l'ensemble de ces inconvénients de l'art antérieur, sous la forme d'un dispositif de prélèvement de tissu d'un animal selon la revendication 1.

L'invention repose ainsi sur une approche nouvelle et inventive du prélèvement et du stockage de tissu d'un animal, permettant de prélever un échantillon sans risquer de le contaminer. En effet, l'élément de coupe et l'échantillon sont insérés directement dans les moyens de stockage, sans intervention extérieure pour d'une part retirer l'échantillon des moyens de prélèvement, et d'autre part insérer l'échantillon dans les moyens de stockage.

De manière astucieuse, les moyens de stockage selon l'invention présentent au moins deux ouvertures. De cette façon, quant bien même l'élément de coupe (ou un emporte-pièce comprenant l'élément de coupe) et l'échantillon sont insérés dans les moyens de stockage et obstruent au moins en partie la première ouverture, il est toujours possible d'ajouter un produit comme un agent conservateur ou un réactif dans les moyens de stockage par l'intermédiaire de la deuxième ouverture, sans avoir à retirer l'échantillon.

De plus, il n'est pas nécessaire selon l'invention de retirer ou découper l'élément de coupe (ou l'emporte-pièce comprenant l'élément de coupe) obstruant la première ouverture pour analyser l'échantillon.

Selon un mode de réalisation particulier, les moyens de stockage comprennent au moins une paroi définissant deux compartiments associés chacun à une desdites ouvertures.

De cette façon, le premier compartiment, associé à la première ouverture, permet de recevoir l'échantillon. Cette première ouverture est obstruée au moins en partie par l'élément de coupe. Le deuxième compartiment, associé à la deuxième ouverture, est quant à lui fermé par des moyens de fermeture amovibles.

La paroi permet ainsi, entre autres, de sécuriser l'échantillon dans le premier compartiment. En effet, grâce à cette paroi, l'échantillon n'est pas accessible à un fraudeur, sans retrait à force, ou découpage, de l'élément de coupe (ou l'emporte-pièce comprenant l'élément de coupe).

Une telle paroi est avantageusement perméable, c'est-à-dire non étanche.

Elle permet notamment le passage de liquide, gaz, ou petits éléments d'un compartiment à l'autre. Ainsi, elle permet le passage d'un réactif, introduit par la deuxième ouverture, du deuxième compartiment vers le premier compartiment. En revanche, elle empêche le passage de l'échantillon localisé dans le premier compartiment vers le deuxième compartiment.

Des billes d'agent conservateur, permettant la dessiccation de l'échantillon, peuvent également être introduites dans le deuxième compartiment.

La paroi perméable permet alors d'empêcher le passage des billes du deuxième compartiment vers le premier compartiment. En effet, il est préférable que ces billes ne soient pas en contact direct avec l'échantillon, afin d'éviter de dénaturer l'échantillon ou des problèmes lors des analyses (pipettes bouchées par exemple).

En revanche, la perméabilité de la paroi permet à l'agent dessiccant d'agir sur l'ensemble du volume du tube, c'est-à-dire dans le premier et le deuxième compartiments, en desséchant l'air ambiant et l'échantillon, qui peut alors se conserver.

Grâce à la deuxième ouverture, il est ainsi possible d'introduire de telles billes dans les moyens de stockage, avant ou après insertion de l'échantillon, et de conserver ces billes dans les moyens de stockage pendant toute la durée de stockage de l'échantillon. Une fois que l'on souhaite analyser l'échantillon, il est possible de retirer ces billes des moyens de stockage, grâce à la deuxième ouverture coopérant avec des moyens de fermeture amovibles. La deuxième ouverture permet donc d'insérer et de retirer ces billes à souhait.

De cette façon, lors de l'analyse de l'échantillon, ces billes ou autre liquide, gaz, ou élément présents dans les moyens de stockage peuvent être retirés, ce qui évite de fausser l'analyse, notamment lorsque ces billes, liquide, gaz, ou autre élément réagissent avec un réactif utilisé.

De plus, le retrait des agents dessiccants permet de réduire la quantité de réactif injectée, le réactif n'étant plus absorbé par ces agents dessiccants.

Par exemple, la paroi comprend un élément appartenant au groupe comprenant :
- une membrane ;
- une grille ;
- une cloison percée d'au moins une ouverture.

Selon un mode de réalisation particulier, les moyens de stockage et/ou de fermeture contiennent au moins un agent dessiccant.

Un agent dessiccant permet notamment d'améliorer la conservation de l'échantillon, en absorbant l'eau contenue dans l'échantillon. Il s'agit par exemple de silicagel (marque déposée) ou plus généralement d'un tamis moléculaire. Différents types de produit à base de silice, d'argile, ou autre, se présentant sous la forme de billes, poudre, gel, solide, liquide, etc, peuvent donc être utilisés.

En particulier, un tel agent dessiccant peut être inséré dans les moyens de fermeture amovible. Ainsi, si ces moyens de fermeture prennent la forme d'un bouchon, des billes d'agent dessiccant peuvent être prévues à l'intérieur de ce bouchon.

Selon une caractéristique particulière de l'invention, les moyens de stockage présentent une forme de révolution, les première et deuxième ouvertures étant situées aux extrémités de la forme de révolution. Par exemple, les moyens de stockage forment un tube ouvert à ses deux extrémités.

En particulier, les moyens de fermeture sont solidarisés aux moyens de stockage de manière réversible, les moyens de stockage comprenant des premiers moyens de solidarisation, et les moyens de fermeture comprenant des seconds moyens de solidarisation complémentaires desdits premiers moyens de solidarisation.

Par exemple, les moyens de fermeture comprennent un bouchon hermétique solidarisé aux moyens de stockage de manière réversible par emboîtement, clippage ou vissage.

Selon un autre mode de réalisation de l'invention, les moyens de prélèvement comprennent un élément de protection amovible disposé dans le prolongement de l'élément de coupe et s'étendant sur au moins une partie substantielle d'un élément formant pointeau d'un outil de prélèvement lorsque les moyens de prélèvement sont solidarisés à l'outil.

Cet élément de protection permet notamment d'éviter la contamination des animaux entre eux lors de l'utilisation du même outil pour le prélèvement de tissu de différents animaux.

Selon un autre aspect de l'invention, le dispositif comprend des moyens anti-retour, conçus façon à permettre le passage d'un échantillon et sa séparation des moyens de prélèvement.

Ces moyens anti-retour peuvent notamment être formés par une rondelle percée en son centre et présentant une pluralité de lamelles souples s'étendant vers ledit centre.

Selon encore un autre aspect de l'invention, les moyens de stockage contiennent un liquide conservateur et/ou réactif.

Dans ce cas, on peut prévoir un opercule d'étanchéité, isolant ledit liquide et apte à être percé lors de l'insertion d'un échantillon.

Il est à noter que ces deux derniers aspects peuvent également être mis en œuvre indépendamment de la présence de deux ouvertures, dans certaines approches.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- les figures 1 et 2 présentent des moyens de stockage d'un échantillon de tissu selon l'art antérieur ;
- les figures 3A et 3B illustrent des moyens de stockage associés à des moyens de fermeture amovibles selon un mode de réalisation de l'invention ;
- la figure 4A illustre une variante des moyens de stockage, comprenant une paroi de séparation ;
- la figure 4B illustre un exemple de paroi ;
- la figure 5 illustre un autre exemple de moyens de stockage, dans lesquels un échantillon est en cours d'insertion ;
- la figure 6 présente les moyens de stockage de la figure 5, l'échantillon étant placé dans un conservateur liquide ;
- la figure 7 est une autre vue des moyens de stockage des figures 5 et 6 ;
- la figure 8 est une vue en coupe des moyens de stockage des figures 5 à 7.

### 5. Description d'un mode de réalisation de l'invention

Le principe général de l'invention repose sur l'utilisation de moyens de stockage d'un échantillon de tissu d'un animal comprenant au moins deux ouvertures.

L'invention propose ainsi un dispositif de prélèvement de tissu comprenant des moyens de prélèvement présentant au moins un élément de coupe destiné à découper et collecter un échantillon de tissu de l'animal, et de nouveaux moyens de stockage de l'échantillon. Une fois collecté, l'échantillon de tissu de l'animal stocké dans les moyens de stockage peut être transmis à un laboratoire pour analyse.

On rappelle que le stockage d'échantillons de tissu d'un animal permet notamment d'identifier ultérieurement l'animal ou de détecter des maladies chez cet animal, au vu d'un examen microscopique ou d'une extraction d'une séquence d'ADN de cellules de l'échantillon par exemple.

On décrit ci-après un mode de réalisation particulier de l'invention, selon lequel les moyens de stockage présentent une forme de révolution définissant un tube. D'autres formes des moyens de stockage peuvent également être envisagées.

Comme illustré en figure 3A, les moyens de stockage 31 selon l'invention présentent au moins deux ouvertures, une première ouverture 32 permettant la réception d'un échantillon, et une deuxième ouverture 33 coopérant avec des moyens de fermeture amovibles 34.

Plus précisément, comme illustré en figure 3B, le tube de stockage 31 selon ce mode de réalisation permet de recevoir l'élément de coupe 35 destiné à découper et collecter un échantillon de tissu de l'animal (ou l'emporte-pièce auquel il appartient), ainsi que l'échantillon prélevé 36, par l'intermédiaire de la première ouverture 32. Avant réception de l'échantillon 36, la première ouverture 32 peut être fermée par un opercule. L'opercule est découpé ou déchiré par l'élément de coupe 35 lors de l'insertion de l'élément de coupe 35 et de l'échantillon 36 dans le tube de stockage 31.

On considère que l'élément de coupe 35 (ou l'emporte-pièce auquel il appartient) présente un diamètre légèrement inférieur à celui du tube de stockage 31. De cette façon, l'élément de coupe 35 obstrue de façon hermétique la première ouverture 32.

En particulier, l'échantillon 36 prélevé peut tomber dans le tube de stockage 31, ou rester coincé dans un logement prévu à cet effet dans l'élément de coupe 35. Un tel logement est ouvert vers l'intérieur du tube de stockage 31, mais fermé vers l'extérieur de ce tube 31.

Ainsi, selon ce mode de réalisation, l'échantillon 36 est prélevé au moyen de l'élément de coupe 35 et assemblé au tube de stockage 31 lors du prélèvement, par exemple par blocage par coincement, sans que l'utilisateur ait besoin de manipuler directement l'échantillon. L'échantillon 36 présent dans le tube de stockage 31 n'est donc pas pollué.

Pour le prélèvement, l'élément de coupe 35 (ou l'emporte-pièce) peut être fixé à une première mâchoire d'un outil de prélèvement comme une pince par exemple, et les moyens de stockage 31 peuvent être fixés à une deuxième mâchoire de l'outil. Les moyens de fermeture 34 peuvent éventuellement être solidarisés aux moyens de stockage 31 avant prélèvement, dans un état fermé, ou bien être solidarisés aux moyens de stockage 31 une fois seulement que le prélèvement a été effectué. Le prélèvement peut être effectué indépendamment de la pose de la marque, ou conjointement à la pose de la marque.

Une fois inséré dans le tube de stockage 31, l'échantillon peut être stocké pour une durée plus ou moins longue, de l'ordre de quelques jours à quelques années. En effet, la première ouverture 32 se trouve fermée hermétiquement (ou quasi-hermétiquement) grâce à l'élément de coupe 35 ou l'emporte-pièce le comprenant, et la deuxième ouverture 33 se trouve fermée hermétiquement ou quasi-hermétiquement) grâce aux moyens de fermeture amovibles 34, prenant par exemple la forme d'un bouchon.

On note à cet effet que les moyens de stockage 31 et les moyens de fermeture amovibles 34 comprennent des moyens de solidarisation complémentaires, comme un filetage et un rainurage. Les moyens de stockage 31 et de fermeture 34 sont par exemple solidarisés de façon réversible par emboîtement, clippage, vissage, etc.

Les moyens de stockage 31 et/ou les moyens de fermeture 34 peuvent contenir un agent conservateur, comme un agent permettant la dessiccation de l'échantillon, dit agent dessiccant. Cet agent peut se présenter sous différentes formes, comme une poudre, un gel, des billes ou perles, etc.

Les moyens de fermeture amovibles 34 selon l'invention permettent ainsi d'évacuer l'agent conservateur avant le traitement de l'échantillon 36. On évite de cette façon que l'agent conservateur réagisse avec un réactif utilisé lors de l'analyse de l'échantillon 36. De plus, l'évacuation de l'agent dessiccant avant le traitement de l'échantillon 36 permet de réduire la quantité de réactif ou produit nécessaire au traitement de l'échantillon. En effet, ce produit ou réactif ne se trouve plus absorbé par l'agent dessiccant.

Selon un mode de réalisation particulier de l'invention, les moyens de stockage 31 comprennent au moins une paroi définissant deux compartiments associés chacun à une des ouvertures.

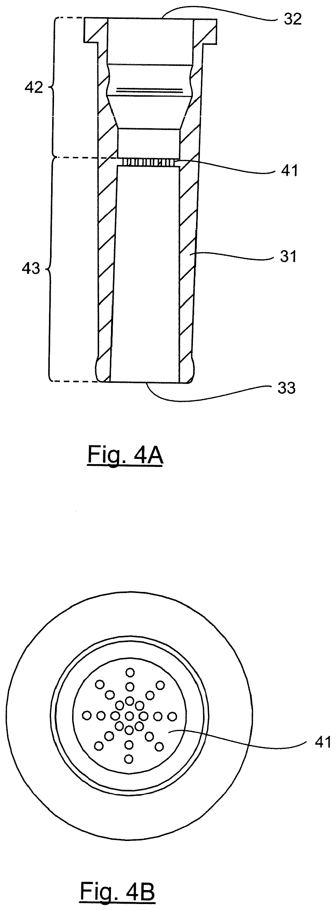

Par exemple, comme illustré en figure 4A, la paroi 41 définit un premier compartiment 42, associé à la première ouverture 32, et un deuxième compartiment 43, associé à la deuxième ouverture 33. La paroi 41 peut être située à différents endroits dans les moyens de stockage 31. La seule contrainte à respecter est quelle ne gêne pas l'insertion de l'élément de coupe 35 et de l'échantillon 36, ou la fermeture de la deuxième ouverture 33. Par exemple, si les moyens de stockage prennent la forme d'un tube, la paroi 41 prend la forme d'un disque, plein ou comprenant une ou plusieurs ouvertures.

Un des rôles de la paroi 41 est de retenir l'échantillon 36 dans le tube de stockage 31, notamment lorsque les moyens de fermeture 34 sont désolidarisés du tube de stockage 31 (état ouvert). De cette façon, l'échantillon 36 est sécurisé dans le premier compartiment 42, et n'est pas accessible à un fraudeur.

En particulier, cette paroi 41 est perméable, au sens où elle peut laisser passer des liquides, gaz, éléments ou particules d'un compartiment à un autre. Il s'agit par exemple d'une membrane, grille, cloison percée d'au moins une ouverture, etc.

De cette façon, il est possible d'injecter un réactif dans le tube de stockage 31 en enlevant le bouchon de fermeture 34, sans avoir à retirer ou découper l'élément de coupe 35 qui obstrue la première ouverture 32, ou à retirer l'échantillon 36.

De plus, la paroi perméable permet d'évacuer un agent dessiccant qui se trouverait dans le premier compartiment 42 avant traitement de l'échantillon. De cette façon et comme indiqué précédemment, on évite une éventuelle réaction du réactif avec l'agent dessiccant, et on limite la quantité de réactif utilisée, puisque celui-ci n'est plus absorbé par l'agent dessiccant.

La paroi 41 permet également de retenir un agent dessiccant qui se trouverait dans le deuxième compartiment 43, afin qu'il n'entre pas en contact direct avec l'échantillon 36 dans le premier compartiment 42. La perméabilité de la paroi permet cependant à l'agent dessiccant d'agir sur l'échantillon 36 pour absorber au moins en partie l'eau qu'il contient.

L'agent dessiccant, présent par exemple sous la forme de billes dans le deuxième compartiment 43, peut être évacué par le bouchon de fermeture 34 avant l'analyse de l'échantillon 36.

La paroi prend par exemple la forme d'une grille ou d'une cloison percée d'au moins une ouverture, comme illustré en figure 4B.

D'autres types de paroi peuvent être envisagés, comme une cloison dure, souple, élastique, en tissu, percée d'une ou plusieurs croix, d'un ou plusieurs trous, etc. Il suffit qu'elle permette le passage d'un réactif pour qu'il puisse atteindre l'échantillon.

Selon une variante, les moyens de fermeture 34 peuvent être réalisés dans un matériau dessiccant, ou l'agent conservateur peut être intégré directement dans les moyens de fermeture 34. Par exemple, l'agent conservateur prend la forme de billes de produit dessiccant localisées entre deux cloisons des moyens de fermeture, une desdites cloisons étant non étanche et venant en contact avec l'intérieur des moyens de stockage.

De cette façon, lorsque les moyens de fermeture 34 sont dans un état ouvert, pour avoir accès à l'intérieur des moyens de stockage 31 et à l'échantillon 36, l'agent conservateur n'est pas à l'intérieur des moyens de stockage, et ne peut pas « fausser » l'analyse de l'échantillon.

Selon une variante de réalisation, les moyens de fermeture peuvent être reliés aux moyens de stockage par l'intermédiaire d'une pièce plastique, d'un filament, etc.

On note également que les moyens de stockage peuvent porter un identifiant (par exemple un numéro ou un code barre). Cet identifiant peut également être reporté sur les moyens de fermeture. Toutefois, du fait que les moyens de fermeture sont amovibles, il est préférable d'indiquer ce numéro directement sur les moyens de stockage. En particulier, lorsque le prélèvement est mis en œuvre conjointement à la pose de la marque, les moyens de stockage portent un identifiant identique à celui figurant sur la partie mâle et/ou femelle de la marque.

### 6. autres aspects

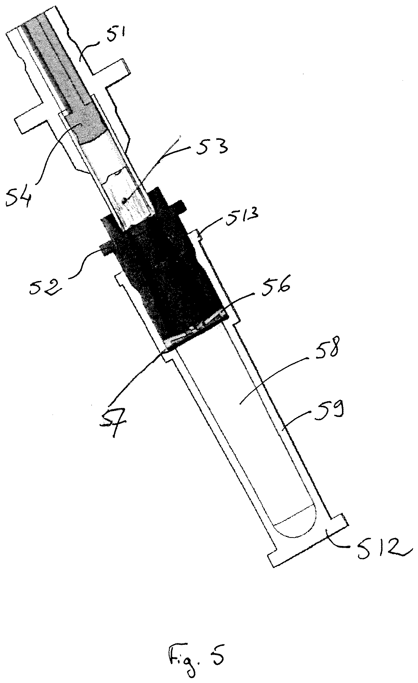

On décrit ci-après, en relation avec les figures 5 à 8, un autre aspect de l'invention, permettant une amélioration de la séparation de l'échantillon et de l'élément de coupe et l'utilisation de moyens de conservation liquides.

On a proposé, dans la demande de brevet FR-08 58453, non publiée, un dispositif de prélèvement de tissu d'un animal comprenant :
- des moyens de prélèvement présentant au moins un élément de coupe destiné à découper un échantillon de tissu de l'animal ; et
- des moyens de stockage de l'échantillon.

Selon cette approche, les moyens de prélèvement comprennent également un élément poussoir mobile par rapport à l'élément de coupe, permettant de pousser l'échantillon dans les moyens de stockage après découpe de l'échantillon par l'élément de coupe.

Cette nouvelle approche du prélèvement d'un échantillon de tissu sur un animal est particulièrement simple et rapide pour l'utilisateur, et ne nécessite pas la pose simultanée d'une marque d'identification. Elle propose en effet d'utiliser deux éléments distincts, mobiles l'un par rapport à l'autre, dont un élément de coupe permettant de découper l'échantillon de tissu, et un élément poussoir permettant de pousser l'échantillon dans les moyens de stockage.

L'utilisation d'éléments distincts pour ces deux opérations présente de nombreux avantages. Par exemple, le fait de découper les tissus puis de pousser l'échantillon dans les moyens de stockage permet d'assurer une bonne découpe de l'échantillon, et d'éviter que des poils restent coincés entre les parois et le bouchon des moyens de stockage. Ces deux éléments distincts permettent également d'obtenir un meilleur prélèvement. En effet, l'élément de coupe n'étant pas destiné à être inséré dans les moyens de stockage, il est possible d'augmenter la taille de l'élément de coupe, c'est-à-dire la longueur de son arête coupante, et donc d'augmenter la taille de l'échantillon prélevé.

Selon un aspect particulier, l'élément poussoir peut comprendre un réservoir contenant au moins un agent séchant et/ou conservateur, sous la forme de poudre ou de granulés. L'utilisation d'un tel agent permet la dessiccation de l'échantillon.

Lors des traitements en laboratoire, le tube est ouvert (extraction de la tête de tube dans laquelle est coincé l'extracteur), et une solution liquide insérée, pour obtenir une réaction, en fonction du type de prélèvement souhaité (recherche de virus, prélèvement d'ADN...).

Un inconvénient de cette approche est qu'il est nécessaire d'attendre un temps plus ou moins long, avant que la réaction s'effectue. En laboratoire, lorsque de très nombreux tubes doivent être traités en parallèle, ces temps d'attente sont complexes à gérer.

Une pipette doit ensuite être insérée dans le tube, pour prélever une partie du liquide, en vue de son analyse. Il apparaît, dans certains cas, que l'échantillon reste collé à la pipette, et/ou à d'autres matériels de laboratoire. Ceci pose, bien sûr, problème, puisque les systèmes sont robotisés, par exemple pour permettre un prélèvement en parallèle sur 96 tubes, selon le format SBS. La présence d'un échantillon extrait par erreur d'un tube peut perturber les traitements, et « polluer » les autres prélèvements effectués simultanément.

Enfin, il apparaît que l'utilisation d'un agent séchant solide n'est pas suffisamment fonctionnelle pour préparer l'échantillon en vue d'une longue conservation, ce qui notamment nécessaire pour les contrôles ADN.

### 6.1 moyens anti-retour

L'invention propose donc, selon un premier aspect, d'équiper le tube de moyens anti-retour de l'échantillon, telle qu'une rondelle à griffes souples.

Lors du prélèvement, l'extracteur passe au travers de cette rondelle, ainsi que l'échantillon. Cet extracteur peut notamment faire partie d'une pince à double mouvement, le premier actionnant l'emporte-pièce pour la découpe de l'oreille, et le second actionnant un extracteur afin de pousser l'échantillon découpé dans le tube, et l'extraire ainsi de l'emporte-pièce.

Au laboratoire, lors de l'ouverture du tube, tel que décrit dans le brevet FR-08 58453 sus-mentionné (on extrait la tête de tube dans laquelle est coincé l'extracteur), l'échantillon reste à l'intérieur du tube, grâce aux griffes de la rondelle. Ainsi, cette rondelle permet des opérations de laboratoire plus sûres. Notamment l'échantillon ne peut pas rester solidarisé aux pipettes ou à d'autres matériels de laboratoire, et ne vient donc pas perturber la suite des traitements.

### 6.2 moyens d'étanchéité

Selon un deuxième aspect de l'invention, on prévoit un operculage à l'intérieur du tube, permettant de conserver un liquide à l'intérieur de celui-ci. Il peut notamment s'agir d'un agent conservateur liquide et/ou d'un liquide permettant une réaction souhaitée en vue du traitement en laboratoire. Cet opercule, par exemple en aluminium, est perforé au passage de l'échantillon et de l'extracteur.

L'insertion de l'échantillon dans cette solution liquide peut permettre, selon les cas, une conservation de longue durée (pour le suivi de l'ADN) et/ou un traitement simplifié en laboratoire, puisque :
- il n'est pas nécessaire, après ouverture du tube, d'insérer un liquide pour obtenir la réaction ;
- il n'est pas nécessaire non plus d'attendre que cette réaction se réalise.

### 6.3 mise en œuvre des deux moyens

Ces deux aspects, d'une part les moyens anti-retour, par exemple sous la forme d'une rondelle souple, et d'autre part les moyens d'étanchéité, peuvent bien sûr être mis en œuvre indépendamment l'un de l'autre. Ils peuvent également être mis en œuvre simultanément, dans un même tube.

Dans ce cas, selon une variante, il est possible qu'un même élément assure deux fonctions, par exemple sous la forme d'un opercule prédécoupé ou présentant des zones de faiblesse et des zones plus résistantes, conçues de façon que, après que l'opercule a été traversé par l'échantillon et l'extracteur, celui-ci puisse assurer la fonction d'anti-retour de l'échantillon.

### 6.4 exemple de mise en œuvre

La technique décrite ci-dessus peut être mise en œuvre dans un dispositif tel qu'illustré par les figures 3A à 4B. Il est également possible de la mettre en œuvre de façon indépendante, comme illustré par les figures 5 à 8.

Comme on le voit sur la figure 5, l'extrémité de la pince, et plus précisément le support emporte-pièce 51, est amenée en contact avec l'extrémité de la tête de tube 52, configurée pour le recevoir. L'échantillon d'oreille 53 découpé se trouve alors dans l'emporte-pièce.

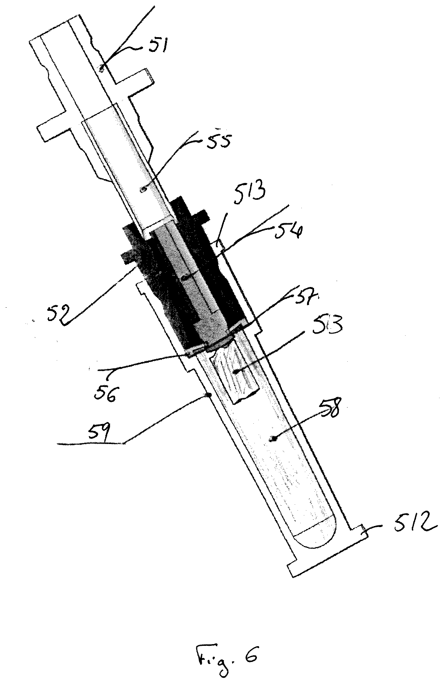

Comme cela apparaît sur la figure 6, l'extracteur 54 coulisse dans la canule 55 et pousse l'échantillon d'oreille 53 dans le tube, au travers de la rondelle anti-retour 56 et/ou de l'opercule d'étanchéité 57.

Dans le cas où un opercule d'étanchéité 57 est présent, on place à l'avance (lors de la fabrication du tube) un liquide 58 conservateur et/ou réactif dans le tube 59, de façon que l'échantillon baigne dans ce liquide dès son insertion.

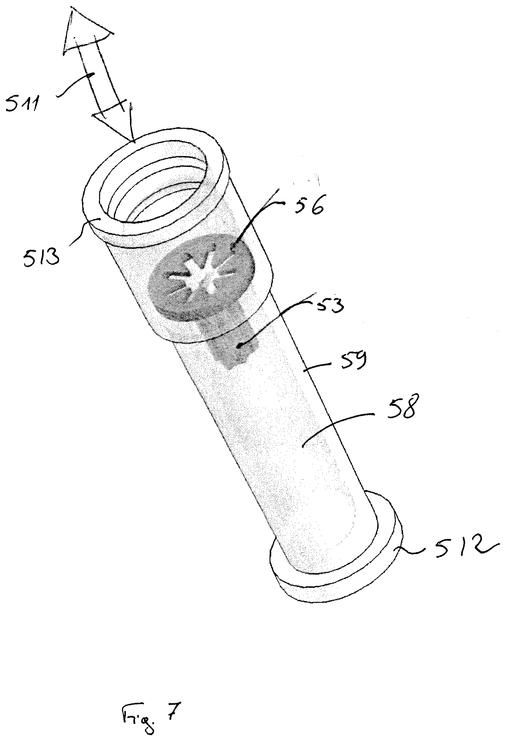

Comme on le voit sur la figure 7, le support emporte-pièce 51 est ensuite retiré, l'extracteur 54 restant présent dans la tête de tube 510, pour fermer le tube (formant donc bouchon). L'extrémité de cet extracteur 54 traverse la rondelle anti-retour 56 de façon à garantir que l'échantillon 53 soit intégralement logé à l'intérieur du tube 59.

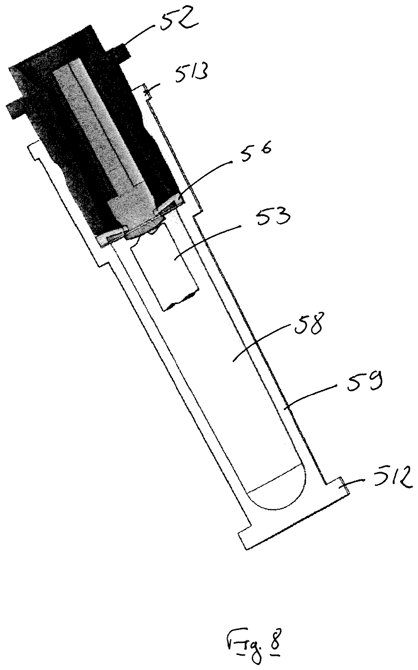

Sur la figure 8, on a retiré la tête de tube 52, en laboratoire, pour permettre les différentes opérations de laboratoire et notamment le pipetage (511).

On distingue, sur cette figure 8, une forme particulière de la rondelle anti-retour 56, présentant une découpe centrale en forme d'étoile, dégageant une zone d'accès central, suffisante pour le pipetage. Les languettes de cette rondelle sont configurées pour empêcher la sortie de l'échantillon 53, même quand celui-ci est accroché, par exemple du fait d'une aspiration, à une pipette.

Le matériau utilisé est donc suffisamment souple pour permettre l'introduction de l'échantillon, et pour empêcher sa sortie. Il est bien sûr possible de prévoir des formes ou des libertés de mouvement spécifiques pour faciliter l'introduction tout en empêchant la sortie.

Par ailleurs, la présence du liquide de conservation 58 dès l'origine permet de conserver suffisamment longtemps l'échantillon 53, y compris en vue d'analyses ADN différées de plusieurs années, sans qu'il soit nécessaire d'extraire l'échantillon pour le placer dans un autre récipient pour sa conservation.

Le liquide conservateur 58 peut comprendre également un ou plusieurs réactifs, de façon que le traitement en laboratoire soit simplifié et accéléré : il suffit alors, en effet, d'ouvrir le tube et d'effectuer le prélèvement (sans qu'il soit nécessaire, après ouverture, d'introduire un réactif liquide, puis d'attendre que la réaction s'effectue avant d'effectuer le prélèvement).

La forme extérieure du tube, et notamment sa base 512 et/ou son col 513, sont avantageusement choisis permettre des traitement par lots, et est par exemple adaptée au format SBS.

Bien sûr, comme expliqué précédemment, cette base 512 est amovible, ou équipée d'un bouchon amovible, permettant le pipetage, le col 513 restant alors obturé par l'extracteur, après prélèvement.

## Revendications

1. Dispositif de prélèvement de tissu d'un animal comprenant :
des moyens de prélèvement présentant au moins un élément de coupe (35) destiné à découper un échantillon de tissu de l'animal et éventuellement un élément poussoir mobile par rapport audit élément de coupe, permettant de pousser ledit échantillon après découpe par ledit élément de coupe,
et des moyens de stockage (31) dudit échantillon dans lesquels ledit élément de coupe ou ledit élément poussoir est inséré après collecte dudit échantillon,
**caractérisé en ce que** le dispositif comprend également des moyens de fermeture amovibles (34) et **en ce que** lesdits moyens de stockage (31) forment un tube présentant deux ouvertures :
- une première ouverture (32) localisée à une première extrémité du tube, permettant la réception dudit élément de coupe (35) ou dudit élément poussoir, et dudit échantillon, ledit élément de coupe (35) ou ledit élément poussoir obstruant ladite première ouverture (32) après son insertion dans celle-ci ; et
- une deuxième ouverture (33) localisée à une deuxième extrémité du tube, distincte de ladite première ouverture (32), et coopérant avec lesdits moyens de fermeture amovibles (34).

2. Dispositif selon la revendication 1, **caractérisé en ce que** lesdits moyens de stockage comprennent au moins une paroi (41) définissant deux compartiments associés chacun à une desdites ouvertures.

3. Dispositif selon la revendication 2, **caractérisé en ce que** ladite paroi (41) est perméable.

4. Dispositif selon l'une quelconque des revendications 2 et 3, **caractérisé en ce que** ladite paroi comprend un élément appartenant au groupe comprenant :
- une membrane ;
- une grille ;
- une cloison percée d'au moins une ouverture.

5. Dispositif selon l'une quelconque des revendications 1 à 4, **caractérisé en ce que** lesdits moyens de stockage (31) contiennent au moins un agent dessiccant.

6. Dispositif selon l'une quelconque des revendications 1 à 5, **caractérisé en ce que** lesdits moyens de fermeture (34) contiennent au moins un agent dessiccant.

7. Dispositif selon l'une quelconque des revendications 1 à 6, **caractérisé en ce que** lesdits moyens de stockage (31) présentent une forme de révolution, lesdites première et deuxième ouvertures étant situées aux extrémités de ladite forme.

8. Dispositif selon l'une quelconque des revendications 1 à 7, **caractérisé en ce que** lesdits moyens de fermeture (34) sont solidarisés auxdits moyens de stockage (31) de manière réversible,
lesdits moyens de stockage comprenant des premiers moyens de solidarisation, et lesdits moyens de fermeture comprenant des seconds moyens de solidarisation complémentaires desdits premiers moyens de solidarisation.

9. Dispositif selon l'une quelconque des revendications 1 à 8, **caractérisé en ce que** lesdits moyens de fermeture (34) comprennent un bouchon hermétique solidarisé auxdits moyens de stockage de manière réversible par emboîtement, clippage ou vissage.

10. Dispositif selon l'une quelconque des revendications 1 à 9, **caractérisé en ce que** lesdits moyens de prélèvement comprennent un élément de protection amovible disposé dans le prolongement dudit élément de coupe et s'étendant sur au moins une partie substantielle d'un élément formant pointeau d'un outil de prélèvement lorsque lesdits moyens de prélèvement sont solidarisés audit outil.

11. Dispositif selon l'une quelconque des revendications 1 à 10, **caractérisé en ce qu'**il comprend des moyens anti-retour, conçus façon à permettre le passage d'un échantillon et sa séparation des moyens de prélèvement, formés par une rondelle percée en son centre et présentant une pluralité de lamelles souples s'étendant vers ledit centre.

12. Dispositif selon l'une quelconque des revendications 1 à 11, **caractérisé en ce que** lesdits moyens de stockage contiennent un liquide conservateur et/ou réactif.

13. Dispositif selon la revendication 12, **caractérisé en ce que** lesdits moyens de stockage comprennent un opercule d'étanchéité, isolant ledit liquide et apte à être percé lors de l'insertion d'un échantillon.

## Patentansprüche

1. Vorrichtung zur Entnahme von Gewebe von einem Tier, umfassend:
Entnahmemittel, die mindestens ein Schneideelement (35) aufweisen, das dazu bestimmt ist, eine Gewebeprobe aus dem Tier herauszuschneiden, und eventuell ein Schiebeelement, das in Bezug auf das Schneideelement beweglich ist, das ermöglicht, die Probe nach dem Herausschneiden durch das Schneideelement zu schieben,
und Mittel zum Speichern (31) der Probe, in die das Schneideelement oder das Schiebeelement nach dem Entnehmen der Probe eingefügt wird,
**dadurch gekennzeichnet, dass** die Vorrichtung auch abnehmbare Verschlussmittel (34) aufweist und dadurch, dass die Speichermittel (31) ein Röhrchen bilden, das zwei Öffnungen aufweist:
- eine erste Öffnung (32), die sich an einem ersten Ende des Röhrchens befindet, die das Aufnehmen des Schneideelements (35) oder des Schiebeelements und der Probe ermöglicht, wobei das Schneideelement (35) oder das Schiebeelement die erste Öffnung (32) verschließt, nachdem es darin eingefügt worden ist, und
- eine zweite Öffnung (33), die die sich an einem zweiten Ende des Röhrchens befindet, die von der ersten Öffnung (32) verschieden ist und die mit den abnehmbaren Verschlussmitteln (34) zusammenwirkt.

2. Vorrichtung nach Anspruch 1, **dadurch gekennzeichnet, dass** die Speichermittel mindestens eine Wand (41) aufweisen, die zwei Kammern bildet, die jeweils einer der Öffnungen zugeordnet sind.

3. Vorrichtung nach Anspruch 2, **dadurch gekennzeichnet, dass** die Wand (41) durchlässig ist.

4. Vorrichtung nach einem der Ansprüche 2 und 3, **dadurch gekennzeichnet, dass** die Wand ein Element aufweist, das zu der Gruppe gehört, umfassend:
- eine Membran,
- ein Gitter,
- eine Trennwand, die mindestens von einer Öffnung durchbohrt ist.

5. Vorrichtung nach einem der Ansprüche 1 bis 4, **dadurch gekennzeichnet, dass** die Speichermittel (31) mindestens ein Trocknungsmittel enthalten.

6. Vorrichtung nach einem der Ansprüche 1 bis 5, **dadurch gekennzeichnet, dass** die Verschlussmittel (34) mindestens ein Trocknungsmittel enthalten.

7. Vorrichtung nach einem der Ansprüche 1 bis 6, **dadurch gekennzeichnet, dass** die Speichermittel (31) eine Rotationsform aufweisen, wobei die erste und zweite Öffnung an den Enden der Form angeordnet sind.

8. Vorrichtung nach einem der Ansprüche 1 bis 7, **dadurch gekennzeichnet, dass** die Verschlussmittel (34) reversibel an den Speichermitteln (31) befestigt sind, wobei die Speichermittel erste Befestigungsmittel aufweisen und wobei die Verschlussmittel zweite Befestigungsmittel aufweisen, die zu den ersten Befestigungsmitteln komplementär sind.

9. Vorrichtung nach einem der Ansprüche 1 bis 8, **dadurch gekennzeichnet, dass** die Verschlussmittel (34) einen dicht schließenden Verschlussstopfen aufweisen, der reversibel an den Speichermitteln durch Einstecken, Einclipsen oder Verschrauben befestigt ist.

10. Vorrichtung nach einem der Ansprüche 1 bis 9, **dadurch gekennzeichnet, dass** die Entnahmemittel ein abnehmbares Schutzelement aufweisen, das in der Verlängerung des Schneidelements angeordnet ist und sich über mindestens einen wesentlichen Teil eines Elements, das die Nadel eines Probenahmewerkzeugs bildet, erstreckt, wenn die Entnahmemittel an diesem Werkzeug befestigt sind.

11. Vorrichtung nach einem der Ansprüche 1 bis 10, **dadurch gekennzeichnet, dass** sie Mittel zur Rückkehrsperre aufweist, die derart ausgebildet sind, um den Durchgang einer Probe und ihr Trennen von den Entnahmemitteln zu ermöglichen, die durch eine Scheibe gebildet sind, die in ihrer Mitte durchbohrt ist und mehrere flexible Lamellen aufweist, die sich in Richtung der Mitte erstrecken.

12. Vorrichtung nach einem der Ansprüche 1 bis 11, **dadurch gekennzeichnet, dass** die Speichermittel eine konservierende und/oder reaktive Flüssigkeit enthalten.

13. Vorrichtung nach Anspruch 12, **dadurch gekennzeichnet, dass** die Speichermittel eine Dichtungskappe aufweisen, die die Flüssigkeit isoliert und beim Einführen der Probe durchbohrt werden kann.

## Claims

1. An animal tissue sampling device comprising:
sampling means having at least one cutting element (35), intended to cut an animal tissue sample, and possibly a push element that is mobile relative to the cutting element, enabling to push the sample cut by the cutting element,
and storage means (31) for storing the sample into which the cutting element or the push element is inserted after the sample has been collected,
**characterized in that** said device also comprises removable closure means (34) and **in that** said storage means (31) forms a tube having two openings:
- a first opening (32) located at a first end of the tube, enabling said cutting element (35) or said push element, and said sample, to be received, in which said cutting element (35) or said push element blocks said first opening (32) after its insertion therein; and
- a second opening (33) located at a second end of the tube, distinct from said first opening (32), and cooperating with removable closure means (34).

2. Device according to claim 1, **characterized in that** said storage means comprise at least one wall (41) defining two compartments each associated with one of said openings.

3. Device according to claim 2, **characterized in that** said wall is permeable (41).

4. Device according to either one of claims 2 and 3, **characterized in that** said wall comprises an element belonging to the group including:
- a membrane;
- a grid;
- a partition perforated with at least one opening.

5. Device according to any one of claims 1 to 4, **characterized in that** said storage means (31) contain at least one drying agent.

6. Device according to any one of claims 1 to 5, **characterized in that** said closure means (34) contain at least one drying agent.

7. Device according to any one of claims 1 to 6, **characterized in that** said storage means (31) have a revolution shape, and the first and second openings are located at the ends of said shape.

8. Device according to any one of claims 1 to 7, **characterized in that** said closure means (34) are reversibly secured to said storage means (31),
said storage means comprising first securing means, and said closure means comprising second securing means complementary to said first securing means.

9. Device according to any one of claims 1 to 8, **characterized in that** said closure means (34) comprise a hermetic seal reversibly secured to the storage means by fitting, clipping or screwing.

10. Device according to any one of claims 1 to 9, **characterized in that** said sampling means comprise a removable protection element arranged in the extension of the cutting element and extending over at least a substantial portion of a rod-forming element of a sampling tool when the sampling means are secured to said tool.

11. Device according to any one of claims 1 to 10, **characterized in that** it comprises anti-return means, designed so as to enable the passage of a sample and the separation thereof from the sampling means, formed by a washer perforated at its center and having a plurality of flexible strips extending toward said center.

12. Device according to any one of claims 1 to 11, **characterized in that** said storage means contain a preservative and/or reactive liquid.

13. Device according to claim 12, **characterized in that** said storage means comprise a sealing lid, isolating said liquid and configured to be perforated when a sample is inserted.
